(19) Europäisches Patentamt

European Patent Office

Office européen des brevets

(11) Veröffentlichungsnummer: **0 355 258 A2**

(12) **EUROPÄISCHE PATENTANMELDUNG**

(21) Anmeldenummer: **89106658.1**

(22) Anmeldetag: **14.04.89**

(51) Int. Cl.4: **G09B 21/00**

(30) Priorität: **22.08.88 DE 3828487**

(43) Veröffentlichungstag der Anmeldung:
**28.02.90 Patentblatt 90/09**

(84) Benannte Vertragsstaaten:
**AT CH DE FR GB IT LI SE**

(71) Anmelder: **Weber, Siegfried**
**Am Weiherberg 11**
**D-7590 Achern(DE)**

(72) Erfinder: **Weber, Siegfried**
**Am Weiherberg 11**
**D-7590 Achern(DE)**

(74) Vertreter: **Zipse & Habersack**
**Lessingstrasse 12**
**D-7570 Baden-Baden(DE)**

(54) **Kommunikations- und Umweltkontrollgerät.**

(57) Die Erfindung betrifft ein Kommunikations- und Umweltkontrollgerät, bestehend aus einer Eingabeeinheit, einer Steuereinheit und Ausgabeeinheiten für die Rückmeldung an den Bediener für die Umweltkontrolle und für die Dokumentation. Die Erfindung ist dadurch gekennzeichnet, daß das Kommunikations- und Umweltkontrollgerät dem Bediener zeitlich nacheinander Zeichen, Zeichengruppen oder Umweltkontrollanweisungen auf einem Anzeigegerät darstellt und der Bediener die gewollten Zeichen, Zeichengruppen oder Umweltkontrollanweisungen durch Betätigen der Eingabeeinheit (2) bestätigt und damit die Weiterverarbeitung, Abspeicherung oder Ausführung bewirkt.

Fig.1

EP 0 355 258 A2

## Kommunikations- und Umweltkontrollgerät

Die Erfindung betrifft ein Kommunikations- und Umweltkontrollgerät, bestehend aus einer Eingabeeinheit, einer Kommunikations- und Steuereinheit und Ausgabeeinheiten für die Rückmeldung an den Bediener für die Umweltkontrolle und für die Dokumentation.

Derartige Geräte werden vornehmlich dazu benutzt, für körperbehinderte Personen, insbesondere mit sehr großer Behinderung, eine Verständigung mit der Umwelt zu ermöglichen oder über die Ausgabeeinheiten des Gerätes auf die Umwelt einzuwirken. Ein wesentliches Merkmal der genannten Geräte besteht darin, Bedienhandlungen, die normalerweise komplexe Bewegungen des menschlichen Körpers erfordern, durch einfachere Bewegungsabläufe zu ersetzen. Durch das rhythmische Drücken eines Knopfes am Gerät können auf diese Weise Mitteilungen an das Pflegepersonal abgegeben werden (Kommunikation), oder es kann das Licht, das Fernsehgerät oder die Pneumatik des Krankenhausbettes bedient werden (Umweltkontrolle).

Die wesentlichste Forderung an ein universell einsetzbares Kommunikations- und Umweltkontrollgerät ist, daß es bei guter Bedienbarkeit durch möglichst einfache Körperbewegungen oder Willensakte gehandhabt werden kann. Beim Einsatz an behinderten Personen soll das Gerät auch bei weiterer Verschlechterung des Gesundheitszustandes noch angewendet werden können, wobei eine ermüdungsfreie Bedienung,eine kurze Einlernzeit und eine leichte Anpassung an die verschiedensten Krankheitsbilder bzw. an die verschiedenen Erfordernisse bei der Umweltkontrolle möglich sein soll. Außerdem wird gefordert, daß das Gerät klein und mobil ist.

Es sind bereits mehrere Lösungen für Kommunikations-und Umweltkontrollgeräte bekanntgeworden, bei denen im wesentlichen als Kommunikations-Steuereinheit ein Personal-Computer oder eine ähnliche Rechneranordnung eingesetzt wird. Die einzelnen Zeichen oder Umweltkontrollanweisungen sind am Bildschirm in Matrixform dargestellt und können durch einen Zeiger, der durch das Betätigen mehrerer Taster oder Sensoren über den Bildschirm bewegt wird, angewählt, abgespeichert oder ausgeführt werden.

Ein System zur visuellen Kommunikation auf der Basis eines Computers besteht in einem Gerät, bei welchem am oberen Teil des Bildschirms das Alphabet und die Zahlen erscheinen. Die gewünschten Buchstaben können angesteuert und mit einem kurzen Impuls produziert werden. Mit einem akustischen Signal kann der Behinderte auf die Mitteilung aufmerksam machen. (Videopac-Computer der Firma Philips)

Ein derartiges Gerät weist jedoch den Nachteil auf, daß alle Buchstaben und Zahlen sowie gegebenenfalls Sonderzeichen, auf dem Bildschirm erscheinen müssen, die dann von dem gegebenenfalls stark körperbehinderten Bediener angesteuert und ausgewählt werden müssen.

Es sind ferner Geräte bekanntgeworden, bei denen die Zeichen oder Anweisungen in Form eines Codes, z.B. des Morsecodes, eingegeben werden. Als Sensoren werden die unterschiedlichsten Typen verwendet, meist jedoch einfache Schalter, die in die Hand genommen und dann per Daumendruck betätigt werden. Hierzu wurden ebenfalls Sensoren bekannt, die auf Saugen und Blasen reagieren, sowie komplexe optische Sensoren, wie z.B. eine Infrarotkamera, die die Augen als Kommunikationsmittel verwenden.

Auch diese Systeme weisen entscheidende Nachteile auf. So ist die Lösung mit der Infrarotkamera mit sehr hohem Hard- und Softwareaufwand verbunden, wobei ein Personal-Computer zwar den Entwicklungsaufwand vereinfachen kann, wobei eine derartige Computereinrichtung aber wegen ihrer äußeren Abmessungen nicht universell einsetzbar ist, da viele Kranke und Behinderte bettlägerig sind und deshalb derartige Installationen am Bett kompliziert sind und vom Benutzer bzw. dessen Pflegepersonal nur schwer durchzuführen sind.

Die Lösung mit dem Morsecode überfordert den Behinderten leicht, da hier mehrere definierte Sensorbetätigungen pro Zeichen notwendig sind. Bei Krankheiten, wie der Ameotropischen Lateralsklerose, sind die Krankheitsverläufe zudem so unterschiedlich, daß die Bedienung derartiger komplexer Geräte äußerst schwierig, wenn nicht gar unmöglich wird.

Der vorliegenden Erfindung liegt daher die Aufgabe zugrunde, ein Gerät der eingangs genannten Art zu schaffen, das einfach und preiswert ist und das auch bei hochgradigen Lähmungszuständen, bei denen nur noch eine Kopfbewegung oder die Atmung möglich ist, eingesetzt werden kann, und das möglichst mit einem einzigen Sensor und mit einem Minimum an Bedienungshandlungen zur Entlastung des Bedieners auskommt.

Zur Lösung der gestellten Aufgabe wird ein Kommunikations- und Umweltkontrollgerät, bestehend aus einer Eingabeeinheit, einer Kommunikations- und Steuereinheit und Ausgabeeinheiten für die Rückmeldung an den Bediener für die Umweltkontrolle und für die Dokumentation vorgeschlagen, welches dadurch gekennzeichnet, ist, daß das Kommunikations- und Umweltkontrollgerät dem Bediener zeitlich nacheinander Zeichen, Zeichengruppen oder Umweltkontrollanweisungen auf einem Anzeigegerät darstellt und der Bediener die gewollten Zeichen, Zeichengruppen oder Umweltkontrollanweisungen durch Betätigen der Eingabeeinheit bestätigt und damit die Weiterverarbeitung, Abspeicherung oder Ausführung bewirkt.

Eine besondere Ausbildungsform des Gerätes gemäß der Erfindung besteht darin, daß das Gerät die Zeichen, Zeichengruppen in anfänglich alphabetischer Reihen folge, durch ein Mikroprozessorsystem gesteuert, zeitlich nacheinander auf ein und derselben Displaystelle eines alphanumerischen Anzeigegerätes darstellt.

Mit dem Kommunikations- und Umweltkontrollgerät gemäß der Erfindung werden mehrere Vorteile erreicht. Durch die zeitlich nacheinander erfolgende Darstellung von Zeichen, Zeichengruppen oder Kontrollanweisungen braucht der Bediener nur im Falle des gewünschten Zeichens od. dgl. die Eingabeeinheit betätigen, und das Gerät bewirkt die Weiterverarbeitung, Abspeicherung oder Ausführung, wobei anschließend das nächste Zeichen zum Vorschlag für den Bediener dargestellt wird. Dadurch ist die Handhabung ohne das Studium einer Bedienungsanleitung vom ersten Augenblick an möglich. Auf diese Weise können die Eingaben zeilenweise automatisch oder per Kommando auf einem angeschlossenen geräuscharmen Drucker ausgegeben werden. Die Bedienung kann durch leichte Kopfbewegungen, die im allgemeinen auch bei schwersten Behinderungen noch möglich sind, erfolgen. Der Bediener kann somit,ohne ein Tastenfeld einer Tastatur zu betätigen, durch zeilenweise Eingaben Briefe beliebiger Länge schreiben. Für den Fall, daß keine Bewegungen des Kopfes möglich sind, können in einfacher Weise andere Sensoreen als Eingabeeinheiten benutzt werden, ohne daß ein für den schwerstbehinderten Bediener unübersichtliches Tableau von Buchstaben und Zeichen gelesen und angesteuert werden muß.

Die Unteransprüche stellen wertvolle Erweiterungen und Bereicherungen des Kommunikations- und Umweltkontrollgerätes dar.

Anhand der Zeichnungen soll am Beispiel von bevorzugten Ausführungsformen das Kommunikations- und Umweltkontrollgerät näher erläutert werden.

Fig. 1 zeigt eine prinzipielle Seitenansicht des Kommunikations- und Umweltkontrollgerätes.

Fig. 2 zeigt eine Ansicht auf das Kommunikations- und Umweltkontrollgerät von oben.

Fig. 3 zeigt eine Atemfrequenzkurve in graphischer Darstellung zur Betätigung eines entsprechenden Atmungssensors.

Fig. 4 zeigt eine Displayanzeige zur Erstellung eines Begriffes durch Sensorbetätigung.

Fig. 5 zeigt am Beispiel einer Displayanzeige den Aufbau von ganzen Sätzen durch Sensorbetätigungen.

Fig. 6 zeigt an einem praktischen Ausführungsbeispiel die Anordnung des Kommunikations- und Umweltkontrollgerätes an einem Krankenbett.

Die Fig. 7, 7a zeigen abgewandelte Ausführungsformen der Anbringung des Kommunikations- und Umweltkontrollgerätes an einem Krankenbett.

Die Fig. 8 und 9 zeigen zwei verschiedene Ansichten mit Schnitt einer anderen Ausführungsform des Kommunikations- und Umweltkontrollgerätes.

Fig. 10 zeigt die Anbringung des Gerätes gemäß den Fig. 8 und 9 an einem Krankenbett.

Die Fig. 11 und 12 zeigen das Erfassen der Kopfbewegung mittels zweier Infrarotsensoren.

Die Fig. 13 und 14 zeigen das Erfassen der Kopfbewegung mittels einer Video-Matrix-Kamera.

Fig. 15 zeigt ein Blockschaltbild des Kommunikations-und Umweltkontrollgerätes gemäß der Erfindung.

Fig. 16 zeigt in Blockdarstellung eine mögliche Software zum Betrieb des Gerätes gemäß der Erfindung.

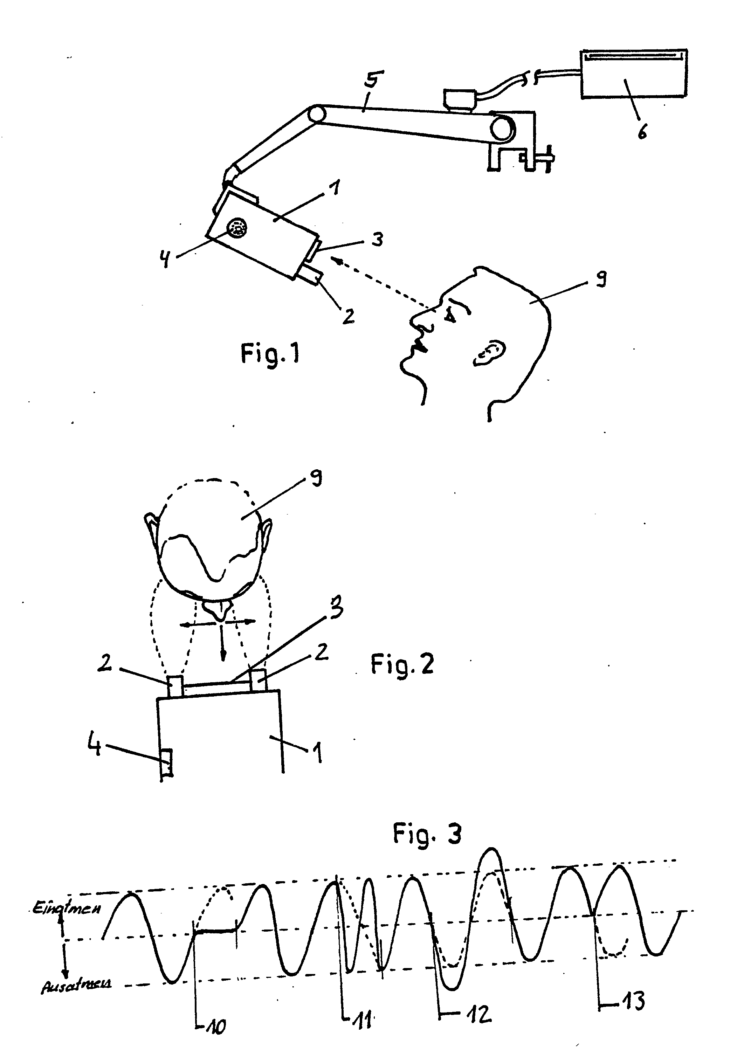

Wie sich aus den prinzipiellen Darstellungen der Fig. 1 und 2 ergibt, besteht das Kommunikations- und Umweltkontrollgerät aus einer Kommunikations-Steuereinheit 1, welche ein Mikroprozessorsystem beinhaltet und welche über eine Eingabeeinheit 2, die ein Sensor oder ein Sensorsystem sein kann, angesprochen wird.

Die Bedienungshandlungen des Gerätes werden gemäß der Bedienerführung über Ausgabeeinheiten an den Bediener akustisch, visuell oder taktil zurückgemeldet. Visuell geschieht dies im folgenden Beispiel über ein alphanumerisches Display 3 und akustisch über einen Lautsprecher 4. Eine taktile Rückmeldung kann über eine nicht dargestellte Hautreizung erfolgen.

Weitere Ausgabeeinheiten, wie weitere Displays, Lautsprecher, Bildschirme usw.,können zu Kontrollanweisungen für weitere Personen, wie z.B. Pflegepersonal, ausgegeben werden. Zu Dokumentationszwecken kann ein Drucker 6 an das Gerät angeschlossen werden. Der Gelenkarm 5 dient der mechanischen Befestigung des Gerätes.

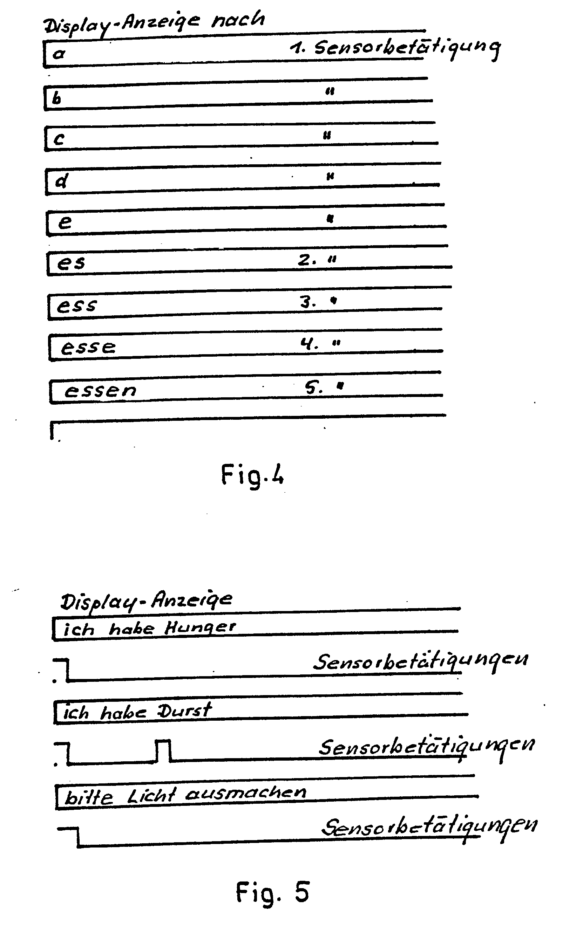

Anhand von Fig. 4 soll die Bedienerführung des Kommunikations- und Umweltkontrollgerätes gemäß der Erfindung erläutert werden. Zunächst ist das Display gelöscht. Wird ein Sensor betätigt, so erscheinen an ein und derselben Stelle bzw. an den selben Stellen zeitlich nacheinander Zeichen oder Zeichengruppen, so lange die Betätigung des Sensors andauert. Ist das vom Bediener gewollte Zeichen auf dem Display dargestellt, so kann dieser die Betätigung beenden, was als Bestätigung gewertet wird. Wenn das zuletzt erschienene Zeichen oder die zuletzt erschienene Zeichengruppe bestätigt wird, wird sie abgespeichert. In dem in Fig. 4 dargestellten Beispiel wurde nach Erscheinen des Buchstabens e die Betätigungshandlung unterbrochen, so daß der Buchstabe e an seiner Stelle stehenbleibt.

Bei der nächsten Sensorbetätigung wurden die Buchstaben so lange angezeigt, bis der Buchstabe s dargestellt wird. Durch Unterbrechung der Betätigung bleibt dieser Buchstabe auf dem Display erhalten,und die Buchstaben an dritter Stelle werden laufend angeboten, bis das zweite Mal der Buchstabe s erscheint, worauf wiederum eine Unterbrechung erfolgt. Dieser Vorgang wird so lange wiederholt, bis ein vollständiges Wort auf dem Display angezeigt wird.

Soll z.B. ein fortlaufender Text geschrieben werden, so gibt das Gerät gemäß der Erfindung die Buchstaben anfänglich in alphabetischer Reihenfolge zeitlich nacheinander auf die Displaystelle.

So können beispielsweise ganze Sätze angezeigt werden, um eine eindeutige Anweisung zu geben. Es ist klar, daß selbst Schwerstbehinderte, die beispielsweise nur noch den Kopf nach rechts und links bewegen können, dieses Verfahren sehr schnell durch Lernvorgänge beherrschen, ohne daß eine Ablenkung durch verhältnismäßig große alphanumerische Tableaus die Bedienung behindert.

Normalerweise benötigt das oben genannte Verfahren bei einer Taktfrequenz von einem Zeichen/Sekunde pro Zeichen eine durchschnittliche Zeit von ca. 11 Sekunden. Diese relativ lange Zeit kann dadurch verkürzt werden, daß das Gerät sich merkt, wie oft ein Zeichen, eine Zeichenfolge oder eine Umweltkontrollanweisung nach einem/einer anderen folgt, um dann später die Zeichen, Zeichengruppen oder Umweltkontrollanweisungen in der Reihenfolge der Wahrscheinlichkeit vorzuschlagen. Dies könnte durch Anlegen von Summier-Speicherzellen gelöst werden, die den jeweiligen Buchstabenkombinationen entsprechen. An einem einfachen Beispiel soll dies verdeutlicht werden.

Nach den Zeichen "sc" ist die Wahrscheinlichkeit, daß ein "h" folgt, sehr hoch. Das Gerät wird also nach den Zeichen "sc" den Buchstaben "h" anbieten. In der jeweiligen Sprache nicht vorkommende Kombinationen werden erst am Schluß auf dem Display dargestellt. Somit braucht, wie in Fig. 5 gezeigt, der Bediener pro Zeichen oder Zeichengruppe nur einmal eine Betätigung durchzuführen, und zwar dann, wenn das dargestellte Zeichen nicht das gewollte ist. Wenn keine genügend hohe Wahrscheinlichkeit vorliegt, wird automatisch normal verfahren (Alphabeth von a nach z darstellen).

Im Idealfall, wenn z.B. die am häufigsten vorkommende Mitteilung an das Pflegepersonal gegeben werden soll, braucht der Bediener nur noch das erste Zeichen im Satz zu wählen. Da ein unbetätigter Sensor Bestätigung signalisiert, werden alle weiteren Zeichen im eingestellten Rhythmus automatisch angefügt. Der zweithäufigste Satz benötigt dann eine zusätzliche Sensorbedienung, der dritthäufigste Satz maximal zwei usw.

Gestoppt werden kann z.B. mit einem Punkt oder einem beliebigen Sonderzeichen. Es ist festzustellen, daß das Gerät sich dadurch auf den jeweiligen Bediener, ungeachtet dessen Sprache und dessen Sprachgewohnheiten, einstellt.

Als Sensoren für die Eingabeeinheit 2 können beliebige Einrichtungen vorgesehen sein, die auf willkürliche Atempausen, Änderungen in der Atemfrequenz, Änderungen in der Atemamplitude oder durch Änderung der Atemphase ansprechen. Dies kann dadurch geschehen, daß z.B. an der Nase oder am Brustkorb des Bedieners als Sensor ein Band mit Dehnungsmeßstreifen angebracht ist, der den Atemrhythmus überwacht. In Fig. 3 ist eine den Atemrhythmus wiedergebende graphische Kurve dargestellt, wobei eine willkürliche Atempause 10 eine Änderung bewirkt und als Betätigung gewertet werden kann.

Auch aus der Änderung der Atemamplitude 12 oder der Atemphase 13 und der Atemfrequenz 11 können weitere Informationen abgeleitet werden.

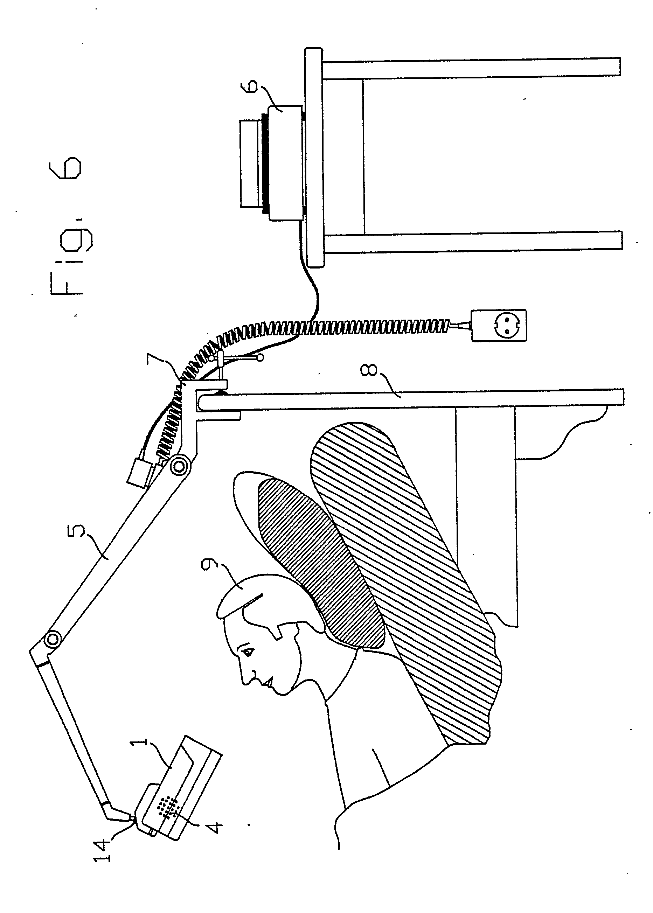

In Fig. 6 ist die Anwendung des Gerätes durch Anbau an ein Krankenbett dargestellt. Der Gelenkarm 5 ist mittels einer Schraubzwinge 7 am rückwärtigen Teil 8 eines Krankenbettes befestigt. Durch ein Kugelgelenk 14 kann das Steuergerät 1 unmittelbar gegenüber dem Kopf 9 des Patienten eingestellt werden.

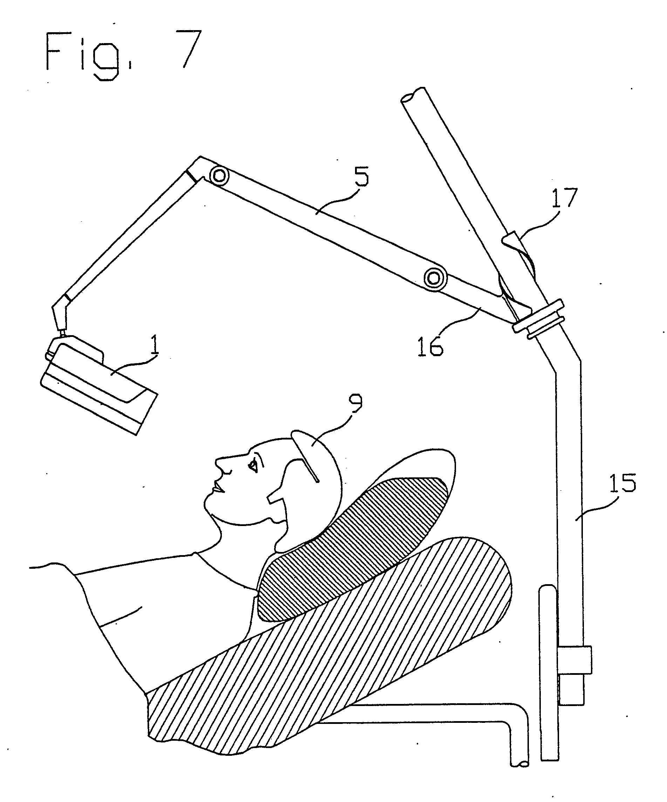

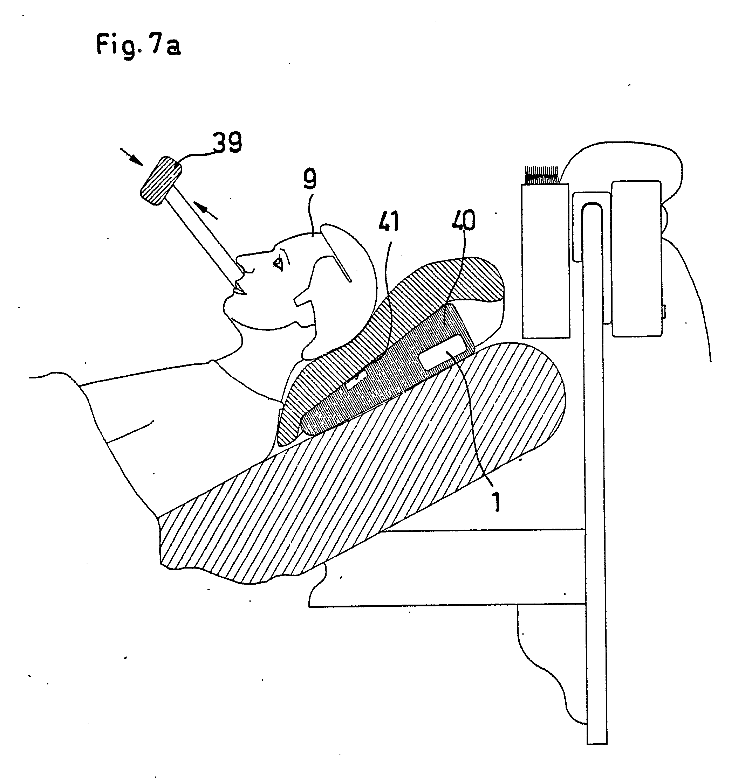

Fig. 7 zeigt eine andere Befestigungsmöglichkeit des Gelenkarmes 5 an einem rohrförmigen Träger 15. Der Gelenkarm 5 ist dabei mit einem Arm 16 gelenkig ver bunden, welcher mit einem Rohrstück 17 in Verbindung steht, das einen mindestens ebenso großen Innendurchmesser aufweist, wie der Rohrträger 15. Das Rohrstück 17 ist seitlich mit einer durchgehenden Öffnung mit einer Öffnungsbreite, die mindestens dem Durchmesser des Rohrträgers 15 entspricht, versehen. Zum Befestigen wird das Rohrstück 17 in den Rohrträger 15 eingehängt. Die Gewichtskraft des Gerätes bewirkt eine Verklemmung zwischen dem oberen und unteren Ende des Rohrstückes 17, wodurch die gesamte Anordnung des Tragarmes 5 und des Gerätes 1 festliegt.

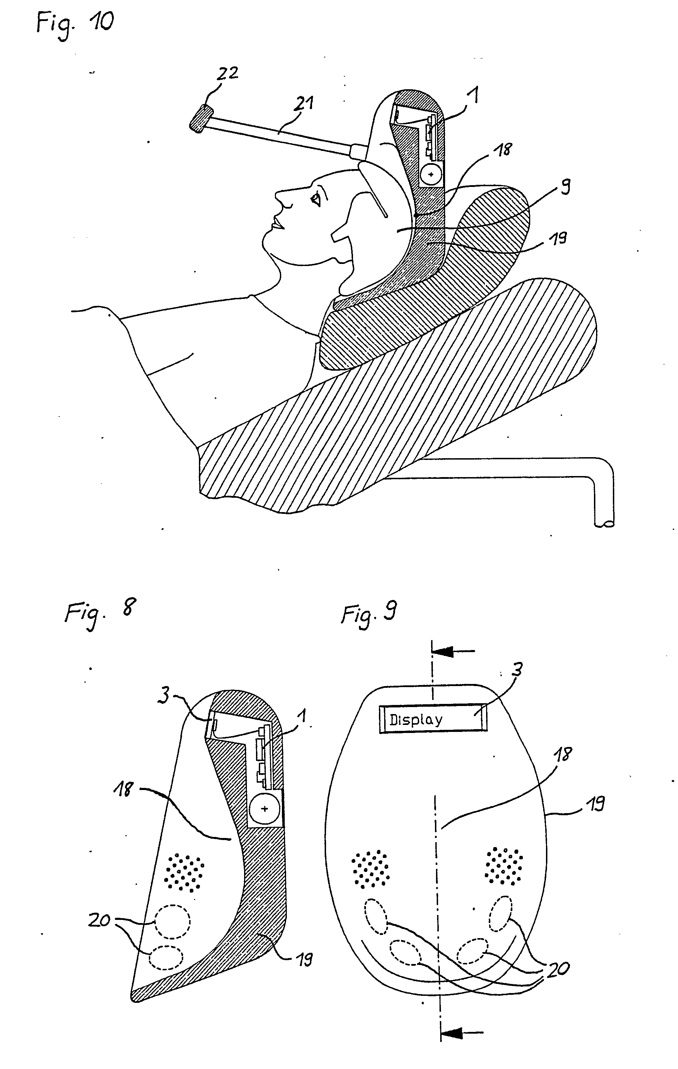

In den Fig. 8, 9 und 10 ist eine mit einer Aussparung 18 für den Kopf 9 versehene Schale 19 vorgesehen, in welcher sich das Steuergerät 1 und,nach oben erscheinend, das Display 3 befinden. Im unteren Bereich der Aussparung 18 können verschiedene, z.B. auf Druck wirkende Betätigungssensoren 20 vorgesehen sein.

Wie sich aus Fig. 10 ergibt, liegt der Kopf 9 des Patienten innerhalb der Aussparung 18. An einem an der Schale 19 angeordneten Arm 21 ist ein weiteres Display 22 vorgesehen, das so eingestellt wird, daß es der Bediener erkennen kann. Durch Betätigung der Sensoren 20, d.h. durch Bewegen des Kopfes nach rechts oder links, können die zuvor angeführten Bedienungsvorgänge ausgeführt werden.

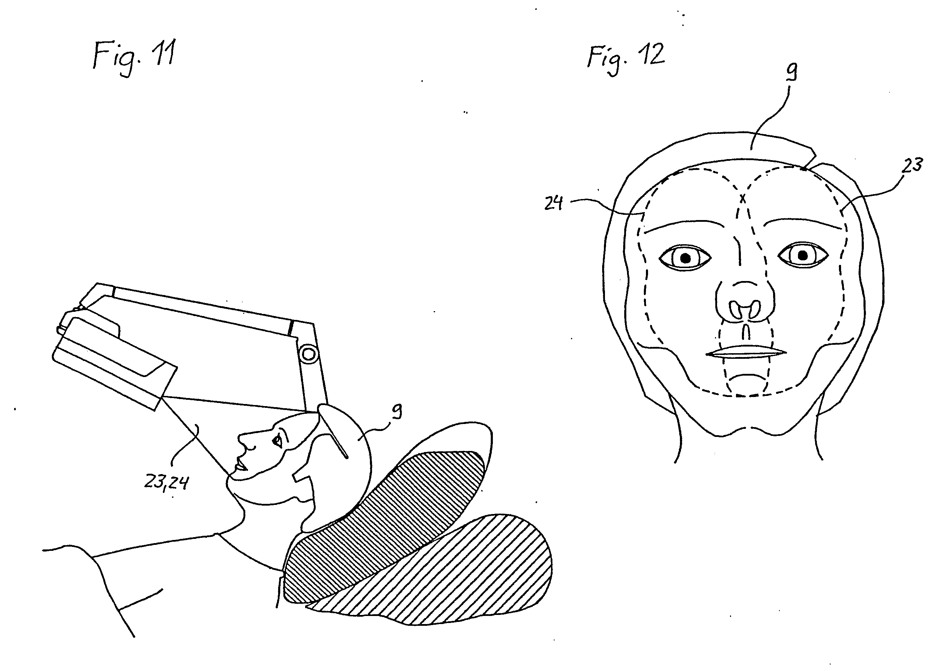

In den Fig. 11 und 12 ist das Erfassen der Kopfbewegung mittels zweier Infrarotsensoren dargestellt. Diese befinden sich im Steuergerät 1 und erzeugen zwei elliptische Kegel 23 und 24, die auf dem Gesicht des Kopfes 9 des Bedieners abgebildet werden. Diese Kegel sprechen auf eine Drehbewegung des Kopfes nach rechts oder links an und lösen die Bedienung aus.

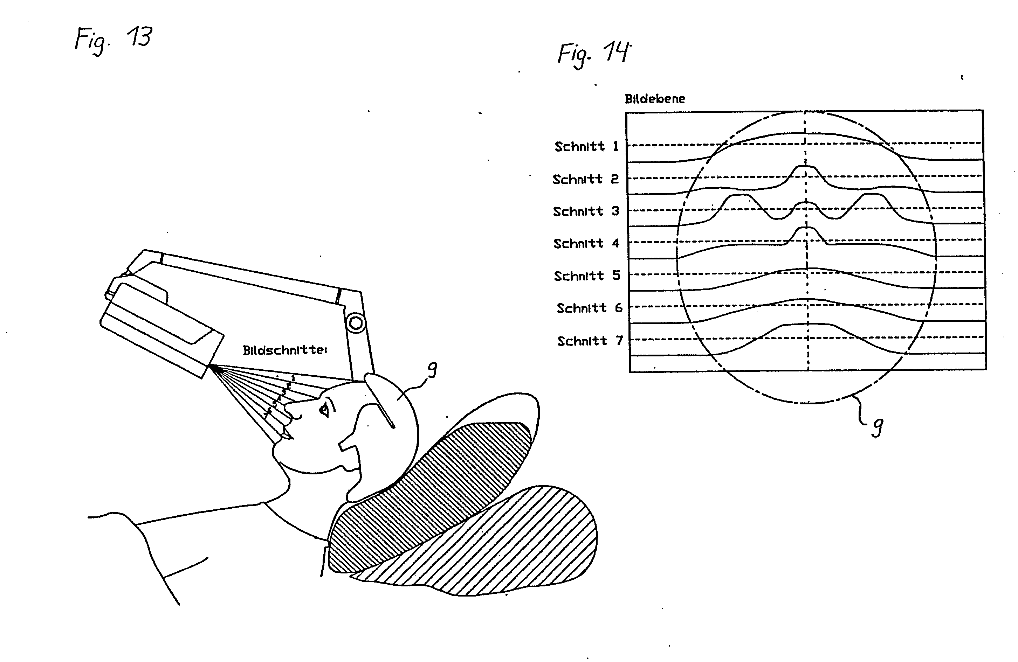

In den Fig. 13 und 14 ist eine andere Möglichkeit der Erfassung der Kopfbewegung mittels einer Video-Matrix-Kamera dargestellt. Die Bildebene ist dabei, wie sich aus Fig. 14 ergibt, z.B. in sieben Bildschnitte unterteilt, längs welcher die Intensitätsverläufe der einzelnen Bildschnitte dargestellt sind. Der gestrichelte Kreis stellt den Kopf des Bedieners dar. Die inneren Erhebungen stellen die Augen, die Nase und den Mund dar. Die Intensitätsverläufe mehrerer Bildschnitte in der Bildebene werden aufgenommen und bei Bewegung des Kopfes durch Veränderung der Intensitätsverläufe die Betätigung der Eingabe in das Gerät gemäß der Erfindung ermöglicht.

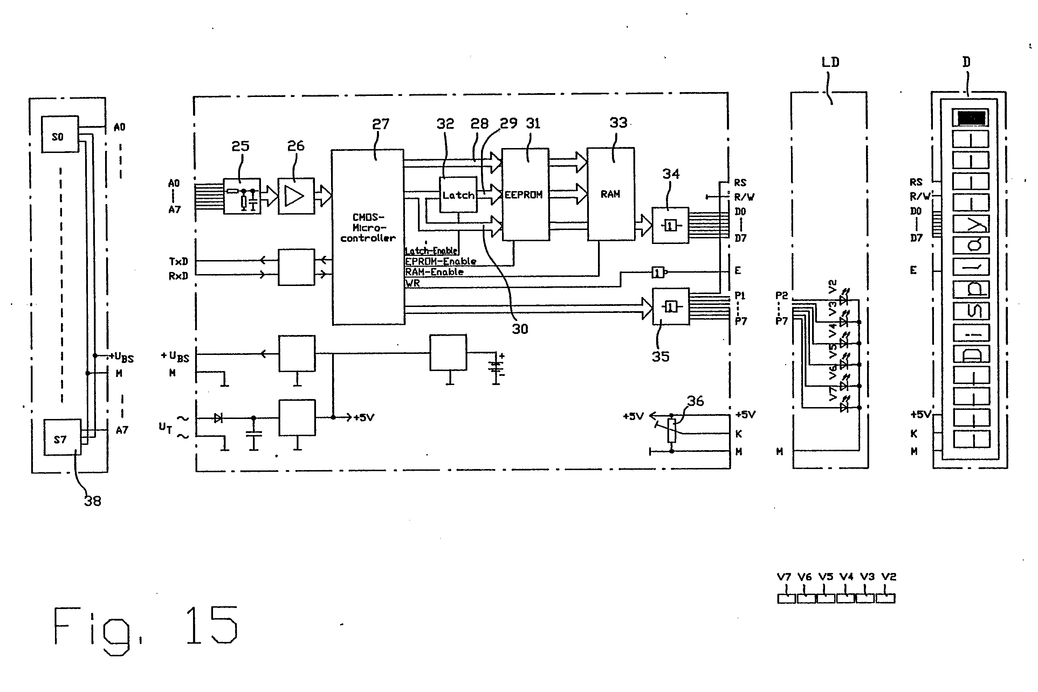

Fig. 15 zeigt ein Blockschaltbild einer bevorzugten Ausführungsform des Kommunikations- und Umweltkontrollgerätes gemäß der Erfindung.

Über die Eingänge A0 bis A7 können bis zu acht Sensoren auf einfache Weise an das Gerät angeschlossen werden, die über Siebglieder 25 und einen Verstärker 26, welcher die Eingangsspannung auf den für den nachfolgenden Analog-Digital-Wandler erforderlichen Pegel bringt, einem CMOS-Microcontroller, z.B. einem Singlechip-Prozessor 8 Bit, zugeführt werden. Der Microcontroller 27 steht über höherwertige Adreßleitungen 28, niederwertige Adreßleitungen 29 und Datenleitungen 30 mit einem EEPROM 31, mit einem RAM-Baustein 33 als flüchtiger Speicher, mit einem Treiberbaustein für die Datenleitungen D0 bis D7, an welche das Display D angeschlossen ist, in Verbindung. Hierbei sind die niederwertigen Adreßleitungen 29 mit einer Latch-Stufe 32 verbunden.

Im EEPROM als elektrisch löschbarer Festwertspeicher sind die Parameter, wie z.B. Wahrscheinlichkeitswerte der Zeichenfolge, remanent abgespeichert und können bei Spannungsausfall nicht verlorengehen. Weiterhin ist es möglich, über Fernleitungen (Telefonmodem) einzugreifen und kundenspezifische Parameter zu ändern.

Das Parallelport des Controllers 27 steht mit einem Treiberbaustein 35 in Verbindung, dessen Datenleitungen P1 bis P7 mit einer Leuchtdiodenanzeige LD in Verbindung steht. Mit einem Potentiometer 36 kann der Kontrast des Displays D eingestellt werden.

Die Sensoren 38 können beliebige, zuvor beschriebene Sensoren sein, wobei jeweils eine entsprechende Anpassung an die Eingänge A0 bis A7 erforderlich ist. Vorzugsweise können kapazitive oder Infrarotsensoren verwendet werden, wobei beide eine analoge Ausgangsspannung liefern. Auch Sensoren mit digitaler Ausgangsspannung sind möglich, jedoch ist dann der Bedienbereich stark eingeschränkt.

Es können analog arbeitende Sensoren mit Schaltcharakteristik oder welche mit digitaler Ausgangsspannung verwendet werden, wobei das Wesentliche der Detektion der Kopfbewegung darin besteht, daß durch Rechts-Links-Bewegung und Mitte drei Zustände erkannt werden und damit drei Informationen abgeleitet werden können. Diese Informationen werden durch Berechnung aus den Sensorsignalen errechnet. Dabei können auch unterschiedliche Größen in Abhängigkeit von der Auslenkung ermittelt werden, wobei die unterschiedliche Größe als weiter Information ausgewertet werden kann, z.B. für die Schnelligkeit der Zeichenfolge auf dem Dislay. Dies hat den Vorteil, daß ein schwerstbehinderter Mensch, der den Kopf nicht so stark bewegen kann, automatisch mit geringerer Geschwindigkeit das Dislay betätigt.

Damit nicht jede kleine Mittenabweichung zu einer Betätigung führt, wird per Software ein Schwellwert gesetzt, der erst überwunden werden muß.

Es kann auch eine Anzeige über die Dioden V2 bis V7 durchgeführt werden, um anzuzeigen, wo sich der Kopf oder eine andere Extremität des Bedieners gerade befindet. Es ist auch vorstellbar, daß die Auslenkanzeige in eine zweite Displayzeile eingeblendet wird.

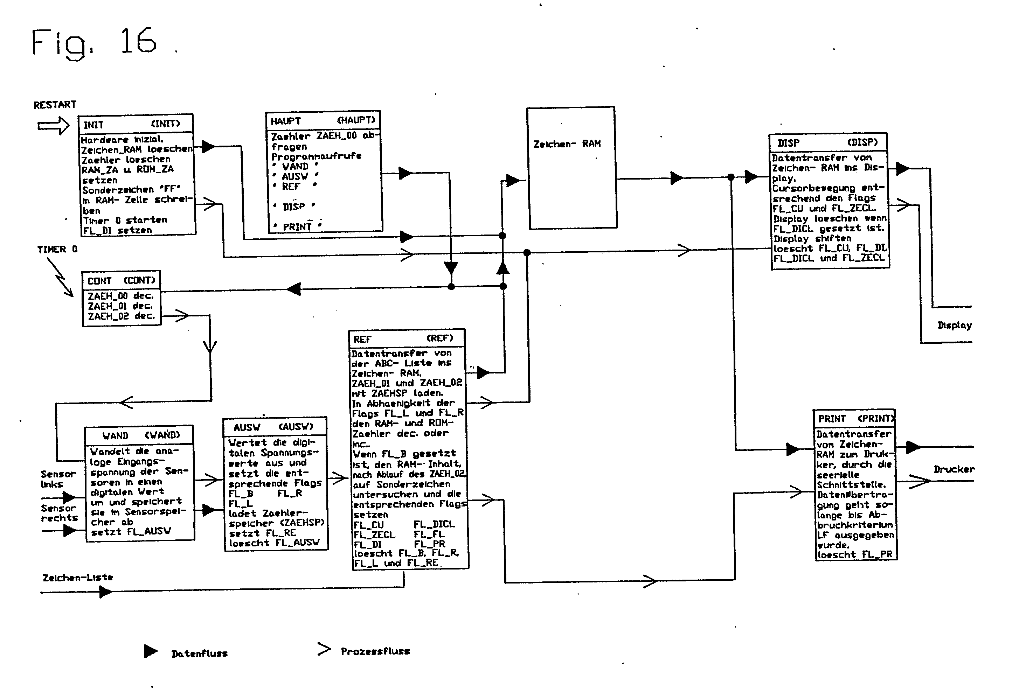

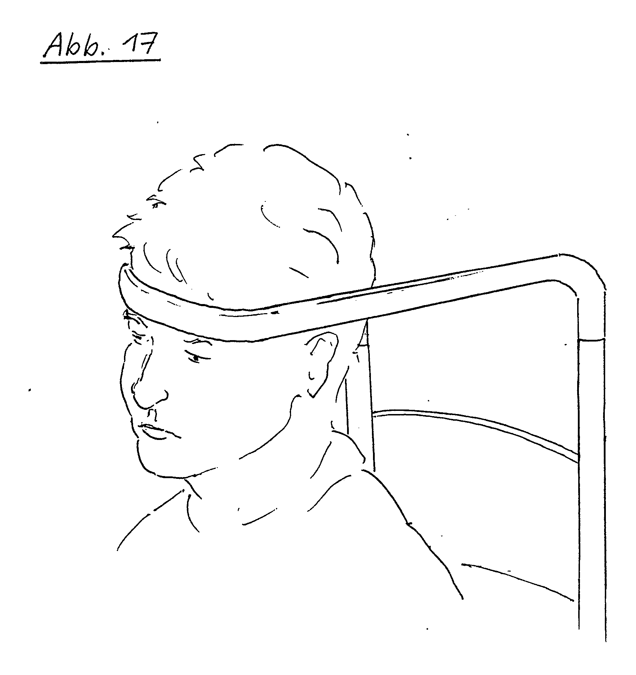

Fig. 16 zeigt in Blockdarstellung eine Möglichkeit der Software zum Betrieb des Gerätes gemäß der Erfindung.

Die Software ist im wesentlichen in ein Initialisierungsprogramm INIT, ein Hauptprogramm HAUPT und Unterprogramme aufgeteilt. Die Unterprogramme bestehen aus
Zählerprogramm CONT
Wandlerprogramm WAND
Auswerteprogramm AUSW
Referenzprogramm REF
Displayprogramm DISP
Printerprogramm PRINT

Das Initialisierungsprogramm INIT legt sämtliche Parameter fest und definiert die Anfangszustände. Ein Hardware-Timer 0, der den benötigten Zeit-Interrupt liefert, wird gesetzt.

Das Hauptprogramm HAUPT ruft, wenn das jeweilige Unterprogramm-Flag gesetzt ist, die einzelnen Unterprogramme auf, und zwar WAND, AUSW, REF, DISP und PRINT.

Das Zählerprogramm CONT bearbeitet drei 8-Bit-Zähler. Die Zähler werden per Interrupt in festen Zeitabständen bei jedem Durchlauf aufgerufen und decrementiert.

Zähler _00 wird im Hauptprogramm HAUPT geladen und ermöglicht den Aufruf des Wandlerprogramms alle 100 ms. Zähler _01 wird im Referenzprogramm REF geladen und bestimmt die Anzeigezeit der einzelnen Zeichen. Zähler _02 wird im Referenzprogramm REF geladen und bestimmt, wie lange die Sensoreinheit ein Signal "Eingabe bestätigen (speichern)" liefern muß, bis das zuletzt angezeigte Zeichen gespeichert wird.

Das Wandlerprogramm WAND wandelt das analoge Eingangssignal der angeschlossenen Sensoren in einen entsprechenden digitalen Wert. Es wird vom Hauptprogramm alle 100 ms aufgerufen. Nach erfolgter Wandlung setzt es ein Flag, das dem Auswerteprogramm signalisiert, daß neue Sensordaten vorliegen und eine Auswertung erfolgen kann.

Das Auswerteprogramm AUSW verarbeitet die im Wandlerprogramm WAND erfaßten Sensordaten. Der Aufruf geschieht durch das Hauptprogramm. Es ermittelt zunächst die Differenz der (beiden) Sensordaten und stellt diese zum Gesamtpegel der Sensoren ins Verhältnis. Ein positives Ergebnis bedeutet, Zeichen oder Zeichenketten in aufsteigender Reihenfolge darstellen, ein negatives Ergebnis bedeutet, die Zeichen oder Zeichenketten in absteigender Reihenfolge darstellen. Minimale positive und negative Ergebnisse werden nicht ausgewertet, damit das System nicht überempfindlich reagiert. Minimale Ergebnisse werden jedoch - wie auch die übrigen -dem Bediener über z.B. Leuchtdioden zu dessen Orientierung rückgemeldet. Das Ergebnis der Berechnung wird für die Weiterverarbeitung den anderen Programmen in Form von Flags zur Verfügung gestellt:
Bestätigungs-/Aufzählen-/Abzählen-Flag.

Das Referenzprogramm REF steuert den Auslegevorgang der Buchstaben aus einer Zeichen/Zeichengruppen-Liste in das RAM. Es wird durch das Hauptprogramm aufgerufen. Nach dem Aufruf wird zunächst ein Datenpointer mit der Listenanfangsadresse geladen. Zähler _01 wird überprüft, ob er abgelaufen ist. Bei nicht abgelaufenem Zähler erfolgt sofort der Rücksprung ins Hauptprogramm. Bei Zählerstand gleich Null werden die im Auswerteprogramm gesetzten Flags überprüft. Bei gesetztem Auf/Abzählen-Flag wird de Inhalt der durch den Pointer definierten ROM-Adresse ins RAM geladen, hiernach der Pointer incrementiert bzw. decrementiert und das Flag gelöscht.

Ein gesetztes Betätigungsflag bewirkt ein Laden des Zählers _02 mit einer Betätigungszeit. Weiterhin wird in diesem Programm der Pointer bezüglich Anfang und Ende der Liste überprüft. Am Ende angelangt, wird er auf den Anfang gesetzt und umgekehrt. Der RAM-Listenpointer wird nach jedem Eintrag incrementiert. Wird ein Maximalwert erreicht, so leitet dies einen automatischen Druckerausdruck (Ausgabeeinheit Drucker) ein. Nach jedem Eintrag wird auch ein Cursor-Flag sowie ein Displayflag gesetzt.

Das Displayprogramm DISP steuert die Ausgabe der ins RAM geschriebenen Zeichen an die Ausgabeeinheit Display. Es wird durch das Hauptprogramm aufgerufen. Ein gesetztes Cursor-Flag setzt den Cursor des Displays eine Stelle weiter nach rechts. Ein als Displayzähler definiertes Byte stellt immer die Position des Cursors im Display dar. Dieser Dislayzähler muß jedesmal überprüft werden, bevor ein weiteres Zeichen ins Display geschrieben wird. Sind bereits alle Stellen beschrieben, so ist eine weitere Ausgabe unzulässig. Ein Wort darf bei 16-stelligem Display maximal nur 15 Buchstaben betragen bzw. es werden lediglich 15 Buchstaben davon dargestellt. Ein volles Display oder die Ausgabe eines vom Bediener eingegebenen Leerzeichens löscht das Display.

Das Printerunterprogramm PRINT steuert die Ausgabe an die Ausgabeeinheit Drucker über die serielle Schnittstelle des Microcontrollers. Es wird durch das Hauptprogramm aufgerufen, falls das Printerflag gesetzt ist. Es liest danach alle ins RAM eingeschriebenen Zeichen/Zeichengruppen auf die serielle Schnittstelle aus. Das RAM wird hiernach wieder gelöscht und für eine neue Eingabe vorbereitet.

Eine weitere Ausführungsmöglichkeit des Kommunikations- und Umweltkontrollgerätes gemäß Fig. 7a besteht darin, daß in einer Kopfunterlage 40 die Sensoren 41 des Gerätes untergebracht werden können, die durch Kopfbewegungen betätigt werden. Es ist ferner möglich, das Steuergerät 1 auch in dieser Unterlage noch unterzubringen.

Seitlich und im Abstand vom Kopf des Bedieners ist ein einstellbares, entsprechend der beiden Pfeile vorder- und rückseitig sichtbares Display 42 angeordnet.

Schließlich kann die Umweltkontrolle über eine serielle Schnittstelle zu weiterer Verarbeitung und Ausführung vorgenommen werden. Diese Umweltkontrolle kann beispielsweise durch Ein- bzw. Ausschalten des Lichtes oder anderer elektrischer und elektronischer Geräte erfolgen.

Ferner ist es möglich, das Display durch einen Bildschirm oder ein anderes Anzeigegerät mit Darstellung der Zeichen in Pixeln zu ersetzen.

Definitionen:

Eine Zeichengruppe ist hier eine Summe von Zeichen die alleine stehend aussagefähig sind: Z. B. eine Summe von Schriftzeichen aller Art, Ziffern, Sonderzeichen wie Punkt, Komma usw.. Es ist darunter aber auch eine zu einem Bild zusammengesetzte Summe einzelner Anzeigesegmente zu verstehen.

Anzeigegeräte sind elektronische Displays und Monitore wie sie in der Elektrotechnik üblich sind.

Die alphabitische Reihenfolge bezieht sich auf die für die jeweilige Schrift gültige, im Anwendungsbereich bekannte Reihenfolge der Zeichen oder Ziffern; bei Ziffern gilt die Reihenfolge der Wertigkeit.

Unter Segment eines Anzeigegerätes ist ein Ausschnitt der Bildfläche zu verstehen.

Auch der Mund ist im Bereich der Nase.

## Ansprüche

Kommunikations- und Umweltkontrollgerät, bestehend aus einer Eingabeeinheit, einer Steuereinheit und Ausgabeeinheiten für die Rückmeldung an den Bediener, für die Umweltkontrolle und für die Dokumentation,
dadurch gekennzeichnet,
daß das Kommunikations- und Umweltkontrollgerät dem Bediener zeitlich nacheinander Zeichen, Zeichengruppen oder Umweltkontrollanweisungen auf einem Anzeigegerät die vorherigen überschreibend bzw. verschiebend darstellt und der Bediener die gewollten Zeichen, Zeichengruppen oder Umweltkontrollanweisungen durch Betätigen der Eingabeeinheit (2) bestätigt und damit die Weiterverarbeitung, Abspeicherung oder Ausführung bewirkt.

2. Kommunikations- und Umweltkontrollgerät nach Anspruch 1,
dadurch gekennzeichnet,
daß das Gerät die Zeichen, Zeichengruppen und Umweltkontrollanweisungen an anfänglich alphabetischer Reihenfolge oder nur in alphabitischer Reihenfolge, vor-oder rückwärts zählend, durch ein Mikroprozessorsystem gesteuert, zeitlich nacheinander auf ein und demselben Segment bzw. auf ein und denselben Segmenten eines Anzeigegerätes darstellt.

3. Kommunikations- und Umwelkontrollgerät nach Anspruch 1 und 2,
dadurch gekennzeichnet,
daß das Gerät die Zeichen, Zeichengruppen oder Umweltkontrollanweisungen in der Reihenfolge der Wahrscheinlichkeit auf dem Anzeigegerät darstellt.

4. Kommunikations- und Umweltkontrollgerät nach Anspruch 1 und 2,
dadurch gekennzeichnet,
daß das Gerät aus der stellenmäßigen und/oder zeitlichen Reihenfolge der bestätigten Zeichen, Zeichengruppen oder Umweltkontrollanweisungen Wahrscheinlichkeitswerte errechnet, die dazu benutzt werden, die Zeichen, Zeichengruppen oder Umweltkontrollanweisungen in der Reihenfolge der Wahrscheinlichkeit auf dem Anzeigegerät darzustellen.

5. Kommunikations- und Umweltkontrollgerät nach einem oder mehreren der vorherigen Ansprüchen,
dadurch gekennzeichnet,
daß das Gerät eine interne Uhr mit Kalender in Hard- oder Softwareausführung besitzt, die nicht nur dazu benutzt wird, die Tages- bzw. Uhrzeit anzuzeigen, sondern daß diese Zeit mit in die Wahrscheinlichkeitsberechnung einbezogen wird oder daß das Gerät einen Umschalter besitzt, durch den die Wahrscheinlichkeit den verschiedenen Erfordernissen während der Tageszeiten angepaßt werden kann.

6. Kommunikations- und Umweltkontrollgerät nach einem oder mehreren der vorherigen Ansprüchen,
gekennzeichnet durch eine an einem Gelenkarm (5) befestigte Kommunikationssteuereinheit (1) mit einem alphanumerischen Display (3), einem Lautsprecher (4) und eine einen Sensor oder ein Sensorsystem aufweisende Eingabeeinheit (2) und eine zusätzliche Ausgabeeinheit.

7. Kommunikations- und Umweltkontrollgerät nach einem oder mehreren der Ansprüche 1 bis 6,
dadurch gekennzeichnet,
daß zwei oder mehrere, analog arbeitende, auf unterschiedliche Entfernung oder Reflexion ansprechende Sensoren vorgesehen sind, deren beide Meßwerte durch das bilden eines Differenz-Signals so zum Gesamtsignal in Beziehung gesetzt werden, daß sich dadurch eine Unterscheidung ergibt, ob ein geänderter Sensor-Bediener-Abstand oder ein Ausweichen des Bedieners nach der Seite hin (Kopfdrehung) vorliegt.

8. Kommunikations- und Umweltkontrollgerät nach einem oder mehreren der Ansprüche 1 bis 6,
dadurch gekennzeichnet,
daß die Eingabeeinheit aus zwei Infrarotsensoren besteht, welche auf die Abbildung der Infrarotkegel auf dem Gesicht des Bedieners ansprechen.

9. Kommunikations- und Umweltkontrollgerät nach einem oder mehreren der Ansprüche 1 bis 6,
dadurch gekennzeichnet,
daß die Eingabeeinheit aus einem oder mehreren kapazitiven Sensor(en) besteht/bestehen, welche(r) geeignet ist/sind eine Kopfbewegung des Bedieners zu erfassen oder daß die Eingabeeinheit aus zwei kapazitiven Sensoren besteht, welche im Mitte-Mitte-Abstand von ca. 20 mm zueinander angeordnet sind und damit mit der Zunge bedient werden können.

10. Kommunikations- und Umweltkontrollgerät nach einem oder mehreren der Ansprüche 1 bis 6,
dadurch gekennzeichnet,
daß die Eingabeeinheit (2) aus einem oder zwei Mikrophon ( en) mit nachfolgendem Frequenzfilter besteht, welche(s) Blas- und Saug-Geräusche in (je) zwei Einzelsignale auflöst.

11. Kommunikations- und Umweltkontrollgerät nach einem oder mehreren der Ansprüche 1 bis 6, dadurch gekennzeichnet,
daß die Eingabeeinheit aus einer Matrix-Kamera oder einer oder mehreren Zeilenkameras besteht, bei welcher/welchen der Intensitätsverlauf bzw. die Intensitätsverläufe eines oder mehrerer Bildschnitte - bei Verwendung einer Matrix-Kamera jedenfalls weniger als deren Zeilenzahl - in der Bildebene aufgenommen und bei Bewegung des Kopfes durch Veränderung der Intensitätsverläufe die Betätigung der Eingabe bewirkt.

12. Kommunikations- und Umweltkontrollgerät nach einem oder mehreren der Ansprüche 1 bis 5, dadurch gekennzeichnet,
daß der Sensor oder die Sensoren des Kommunikations- und Umweltkontrollgerätes oder die Sensoren zusammen mit der Steuereinheit am Hinterkopf oder Nackenbereich des Bedieners positioniert sind.

13. Kommunikations- und Umweltkontrollgerät nach einem oder mehreren der Ansprüche 1 bis 5, dadurch gekennzeichnet,
daß das Kommunikations- und Umweltkontrollgerät in einer dem Kopf angepaßten und ihn teilweise aufnehmenden Schale untergebracht ist, wobei im Bereich der Ohren ein oder mehrere Lautsprecher oder sonstige Schallerzeuger angebracht sind.

14. Kommunikations- und Umweltkontrollgerät nach einem oder mehreren der Ansprüche 1 bis 5, dadurch gekennzeichnet,
daß der Sensor oder die Sensoren des Kommunikations-und Umweltkontrollgerätes oder die Sensoren zusammen mit der Steuereinheit in einer Kopfunterlage am Hinterkopf des Bedieners positioniert sind.

15. Kommunikations- und Umweltkontrollgerät nach einem oder mehreren der Ansprüche 1 bis 5, dadurch gekennzeichnet,
daß der Sensor oder die Sensoren des Kommunikations-und Umweltkontrollgerätes oder die Sensoren zusammen mit der Steuereinheit in einer Kopfunterlage am Hinterkopf des Bedieners positioniert sind und diese Kopfunterlage ein Kopfkissen ist.
Anmerkung: Die Kopfunterlage kann hierbei auch so geartet sein, daß eine Bedienung in der Seitenlage (Ohr liegt auf der Unterlage) möglich ist. Wesentlich ist, daß nicht von vorn, sondern von unten oder hinten her die Kopfbewegung registriert wird.

16. Kommunikations- und Umweltkontrollgerät nach einem oder mehreren der Ansprüche 1 bis 5,

dadurch gekennzeichnet,
daß das Kommunikations- und Umweltkontrollgerät in einer dem Kopf angepaßten und ihn teilweise aufnehmenden Schale untergebracht ist, wobei im Hinterkopfbereich, im Nackenbereich oder seitlich am Kopf Sensoren vorgesehen sind, die auf eine Drehbewegung des Kopfes nach rechts oder links ansprechen und die Bedienung ermöglichen.
Anmerkung: Auch in der Seitenlage kann auf diese weise eine Kopfbewegung ausgewertet werden.

17. Kommunikations- und Umweltkontrollgerät nach Anspruch 12,13,14,15 oder 16, dadurch gekennzeichnet,
daß an der Kopfunterlage ein vom Bediener einsehbares Display oder eine sonstige Anzeigeeinheit befestigt ist.

18. Kommunikations- und Umweltkontrollgerät nach einem oder mehreren der Ansprüche 1 bis 5, dadurch gekennzeichnet,
daß die Sensoren symmetrisch an oder in einem Rahmen im bereich der Stirn oder im Bereich der Schläfen befestigt sind, der gleichzeitig als Kopfstütze dienen kann (17) und der an einem Tisch oder an einem (Roll-) Stuhl befestigt wird.

19. Schaltungsanordnung für ein Kommunikations- und Umweltkontrollgerät nach einem oder mehreren der vorhergehenden Ansprüche, dadurch gekennzeichnet,
daß über Siebglieder (25) und einen Verstärker (26) die Eingänge (A0 bis A7) an einen CMOS-Microcontroller (27), z. B. einen Singlechip-Prozessor (8 Bit), geschaltet sind, der über höherwertige Adreßleitungen (28), niederwertige Adreßleitungen (29), diese über ein Latch (32) und Datenleitungen (30) mit einem EEPROM (31), einem RAM-Baustein (33) und einem Treiberbaustein (34), an welchen das Display (D) angeschlossen ist, in Verbindung steht, wobei ferner der Microcontroller (27) mit einem Treiberbaustein (35) in Verbindung steht, dessen Datenleitungen (P1 bis P7) mit einer Leuchtdiodenanzeige (LD) in Verbindung steht, wobei mit einem Potentiometer (36) der Kontrast des Displays eingestellt werden kann, und daß die Sensoren (38), die beliebige Sensoren, selbst einfache Schalter, insbesondere aber kapazitive oder Intrarot-Sensoren mit analogen Ausgangsspannungen sein können, an die Eingänge (A0 bis A7) angeschlossen sind.

20. Software zum Betrieb eines Kommunikations- und Umweltkontrollgerätes gemäß Anspruch 20, gekennzeichnet durch
ein Initialisierungsprogramm (IN) zur Festlegung sämtlicher Parameter und zur Definition der Anfangszustände, ein Hauptprogramm (HAUPT) zum Aufruf von insbesondere fünf Unterprogrammen. Dies sind: Ein Wandlerprogramm (WAND) zur Umwandlung der analogen Eingangssignale der angeschlossenen Sensoren in den entsprechenden digitalen Wert, ein Auswerteprogramm (AUSW) zur Verarbeitung der im Wandlerprogramm (WAND) erfaßten Sensordaten, wobei dieses zunächst die Differenz der Sensordaten ermittelt und diese zum addierten Gesamtpegel der Sensoren ins Verhältnis setzt, ein Referenzprogramm (REF) zur Steuerung des Auslesevorganges der Buchstaben aus einer Zeichen/Zeichengruppen/Umweltkontrollanweisungs-Liste ins RAM (RAM), ein Displayprogramm (DISP) zur Steuerung der Ausgabe der in das RAM (RAM) eingeschriebenen Zeichen, Zeichengruppen oder Umweltkontrollanweisungen an die Ausgabeeinheit Display und ein Printerprogramm (PRINT), welches die Ausgabe an die Ausgabeeinheit Drukker über eine serielle Schnittstelle oder parallele Schnittstelle steuert,
weiterhin gekennzeichnet durch ein Zählerprogramm (CONT) zur Bearbeitung von drei 8-Bit-Zählern.

Fig. 1

Fig. 2

Fig. 3

Einatmen

Ausatmen

Display-Anzeige nach

| a | 1. Sensorbetätigung |
| b | " |
| c | " |
| d | " |
| e | " |
| es | 2. " |
| ess | 3. " |
| esse | 4. " |
| essen | 5. " |

Fig. 4

Display-Anzeige

| ich habe Hunger |
Sensorbetätigungen

| ich habe Durst |
Sensorbetätigungen

| bitte Licht ausmachen |
Sensorbetätigungen

Fig. 5

Fig. 6

EP 0 355 258 A2

Fig. 7

Fig. 7a

Fig. 10

Fig. 8

Fig. 9

Display

Fig. 12

Fig. 11

Fig. 13

Fig. 14

Bildebene

Schnitt 1
Schnitt 2
Schnitt 3
Schnitt 4
Schnitt 5
Schnitt 6
Schnitt 7

Bildschnitte

9

EP 0 355 258 A2

EP 0 355 258 A2

Fig. 15

LD

D

25 26 27 32 28 29 31 33

34 35 36 38 30

CMOS-Microcontroller

Latch

EEPROM

RAM

Latch-Enable
EPROM-Enable
RAM-Enable
WR

A0
A7

TxD
RxD

+U_BS
M

U_T ~
~

+5V

A0

S0

S7

+U_BS
M

A7

RS
R/W
D0
D7

E

P1
P7

+5V
K
M

P2
P7

V7 V6 V5 V4 V3 V2
M

RS
R/W
D0
D7

E

+5V
K
M

display

V7 V6 V5 V4 V3 V2

# Fig. 16 .

RESTART

INIT        (INIT)

Hardware inizial.
Zeichen_RAM loeschen
Zaehler loeschen
RAM_ZA u. ROM_ZA
setzen
Sonderzeichen "FF"
in RAM- Zelle schreiben
Timer 0 starten
FL_DI setzen

HAUPT        (HAUPT)

Zaehler ZAEH_00 abfragen
Programmaufrufe
' WAND '
' AUSW '
' REF '

' DISP '

' PRINT '

Zeichen- RAM

DISP        (DISP)

Datentransfer von
Zeichen- RAM ins Display,
Cursorbewegung entsrechend den Flags
FL_CU und FL_ZECL.
Display loeschen wenn
FL_DICL gesetzt ist.
Display shiften
loescht FL_CU, FL_DL
FL_DICL und FL_ZECL

TIMER 0

CONT (CONT)

ZAEH_00 dec.
ZAEH_01 dec.
ZAEH_02 dec.

REF        (REF)

Datentransfer von
der ABC- Liste ins
Zeichen- RAM.
ZAEH_01 und ZAEH_02
mit ZAEHSP laden.
In Abhaenigkeit der
Flags FL_L und FL_R
den RAM- und ROM-
Zaehler dec. oder
inc..
Wenn FL_B gesetzt
ist, den RAM- Inhalt,
nach Ablauf des ZAEH_02
auf Sonderzeichen
untersuchen und die
entsprechenden Flags
setzen
FL_CU        FL_DICL
FL_ZECL        FL_FL
FL_DI        FL_PR
loescht FL_B, FL_R,
FL_L und FL_RE.

Display

WAND (WAND)

Wandelt die analoge Eingangsspannung der Sensoren in einen
digitalen Wert
um und speichert
sie in Sensorspeicher ab
setzt FL_AUSW

AUSW (AUSW)

Vertet die digitalen Spannungswerte aus und
setzt die entsprechende Flags
FL_B        FL_R
FL_L
ladet Zaehlerspeicher (ZAEHSP)
setzt FL_RE
loescht FL_AUSW

PRINT (PRINT)

Datentransfer
von Zeichen-
RAM zum Drukker, durch die
seerielle
Schnittstelle.
Datenübertragung geht solange bis Abbruchkriterium
LF ausgegeben
wurde.
loescht FL_PR

Sensor
links

Sensor
rechts

Drucker

Zeichen-Liste

▶ Datenfluss          > Prozessfluss

EP 0 355 258 A2

Abb. 17